# EUROPEAN PATENT APPLICATION

(11) **EP 4 656 199 A1**
(43) Date of publication of application: **03.12.2025**
(21) Application number: 25175679.7
(22) Date of filing: 12.05.2025
(51) Int. Cl.: A61K 36/53, A61K 36/899, A23L 33/105, A61P 25/20, A61P 25/22

(54) **POWDER COMPOSITION BASED ON LAVENDER OIL EXTRACT AND RICE OKARA VEHICLE**

(30) Priority: 31.05.2024 IT 202400012478
(71) Applicant: Gricar Chemical S.r.l., 20861 Brugherio MB (IT)
(72) Inventor: Carenzi, Gian Marco, 20861 Brugherio MB (IT)
(74) Representative: Perani & Partners S.p.A.

(57) **Abstract**

The object of the invention relates to a powder composition comprising a Lavender oil extract, comprising in turn linalool and linalyl acetate, each in an amount comprised between 20% and 45% by weight based on the total weight of the extract, and an active and carrier agent consisting of rice okara, for use in treating mild insomnia and/or mild anxiety.

## Description

### FIELD OF THE INVENTION

The field of the present invention relates to a technology for carrying the Lavander oil extract, which is for use in treating mild insomnia and/or mild anxiety. The technology being discussed consists of a composition in powder form comprising the Lavender oil extract effectively carried by a solubilizer and stabilizer, i.e. rice okara; this latter also acts as an active, thereby increasing and/or assisting the intrinsic therapeutical efficacy of the Lavender oil extract.

### BACKGROUND ART

The flowers and flowering tops of *Lavandula angustifolia* are known for containing active ingredients, especially linalool and linalyl acetate, useful for promoting relaxation and sleep.

From the flowers and flowering tops of Lavender, an essential oil is obtained by steam current, which can be variously concentrated in these actives. The most effective essential oils are those with a higher linalool and linalyl acetate content.

According to the European Pharmacopoeia monograph on Lavender essential oil, the linalool and linalyl acetate content is as follows: linalool from 20.0% to 45.0%; linalyl acetate from 25.0% to 47.0%.

Traditional herbal drugs on the market contain Lavender essential oil complying with the parameters set out in the Pharmacopoeia monograph and are indicated for the relief of mild anxiety symptoms and for promoting sleep.

For these medicines, the recommended dosage for adults and adolescents over 12 years of age is one form dose containing 80 mg of Lavender oil (*Lavandula angustifolia Miller, aetheroleum*), to be taken once daily with water.

The duration of treatment is two weeks, but if symptoms persist, a doctor or pharmacist should be consulted.

Possible side effects are mild and have a frequency that cannot be estimated from the available data. These are, at most, mild gastrointestinal discomfort (e.g., belching) and allergic skin reactions in case of individual hypersensitivity.

Lavender contains a series of active compounds, including linalool and linalyl acetate, that have been studied for their effects on the central nervous system. Indeed, these compounds can interact with neurotransmitters in the brain to help reduce agitation and promote relaxation.

Specifically, one of the main ways Lavender oil can act is through interaction with the neurotransmitter GABA or gamma-aminobutyric acid. This is an inhibitory neurotransmitter playing a key role in reducing the neuronal activity in the brain. When GABA levels are high, it tends to reduce anxiety and stress feelings. Some studies suggest that Lavender oil can increase GABA activity in the brain, inducing a calming and anxiolytic effect.

In addition, research suggests that Lavender oil can also interact with the receptors of serotonin, another neurotransmitter involved in mood and anxiety regulation. Some studies suggest that Lavender oil can increase serotonin levels in the brain, which can have an antidepressive and anxiolytic effect.

Lavender essential oil also acts by reducing levels of cortisol, i.e. the stress hormone. Reducing the presence of this hormone can help reduce anxiety and stress feelings.

All these properties provide a good relaxing action and a sedative effect, which can facilitate falling asleep and enhance the quality of sleep. Indeed, some studies suggest that the Lavender essential oil can increase the amount of deep sleep an individual gets.

### Lavender essential oil as an ingredient in food supplements

Lavender essential oil can be useful as an active ingredient in food supplements able to promote the natural physiological functions related to sleep and relaxation.

The use of plant extracts and preparations (*"botanicals"*) in food supplements is currently regulated by the Decree of the Ministry of Health, August 10th, 2018. Annex 1 of this Ministerial Decree lists permitted plants and parts thereof, accompanied where necessary by additional directions for use. This list is applicable only to plants/parts of plants and derivatives thereof (e.g., extracts or other preparations) having a significant history of consumption as a food before 1997. Annex 1 has been subjected to a new amendment by Directorial Decree n. 12, December 13th, 2023, intended to specify the meaning of the indication "*aetheroleum*" in the column "traditionally used part" in order to properly identify the part that can be used in food supplements by correlating it to the significant use as defined by Regulation No. 2015/2283.

Lavender essential oil obtained from flowers and tops of the plant *Lavandula angustifolia Mill.*, is included in the list with the following description:

| | |
|---|---|
| *Botanical name* | *Lavandula angustifolia Mill.* |
| *Family* | *Lamiaceae* |
| *Synonyms* | *Lavandula angustifolia subsp. angustifolia, Lavandula officinalis Chaix* |
| *Traditionally used part* | summitas cum floribus, flos, aetheroleum* (*essential oil obtained from the part of the plant indicated in Annex 1 of the Ministerial Decree, August 10th, 2018) |
| *Ministerial reference guidelines for physiological effects* | flos, summitas cum floribus, aetheroleum*: relaxation (sleep; in case of stress). Normal mood tone |
| *Function* | Digestive function; regulates gastrointestinal motility and gas elimination |

### Prior art problem

In general, oil extracts (or essential oils) are notorious for being *physically* and/or *chemically* unstable. Oil extracts can be *physically* unstable because they possess substances or associations of substances which are volatile in nature and, therefore, easily tend to disperse. On the other hand, oil extracts can be *chemically* unstable because, as in some cases, they contain substances having more or less easily labile, even though covalent, bonds.

A manner for overcoming these limitations and protecting essential oils normally consists in the encapsulation in carrier systems allowing to increase the bioavailability and to improve their physico-chemical stability, while reducing their volatility/ toxicity. The most common carrier systems are micro- and nano-emulsions, liposomes and lipid nanoparticles, which are all systems in liquid or semi-solid form. These technologies are more complex systems and require fine preparation methods.

Further, on the market, some nutrients or agri-food residues are known, such as rinds and peels, unripe or damaged fruits, seeds, peels, exhausted pulp, which can serve as *carriers* or vehicles of oil extracts.

It is also true, however, that agri-food residues or food products differ from each other, for example in terms of nutrients (polysaccharides, lipids, and fats), or according to the physico-chemical nature thereof and the technological processes they may undergo. The same applies, on the other hand, to the oil extracts to be carried.

As far as the Lavender essential oil is specifically concerned, it has limits of use, in particular it is hydrophobic in nature and contains unstable and/or volatile actives which tend to disperse, besides containing potentially toxic substances. The main factors affecting the stability are the volatilization and/or degradation due to temperature, light and oxygen availability.

Lavender oil extract contains a high content of linalyl acetate, in addition to many other compounds which are volatile and lipophilic in nature and belong to different chemical classes. The components included in the Lavender essential oil tend to decrease in amount due to the volatilization and/or conversion and/or degradation reactions thereof, which results in a partial or full loss of the pharmacological properties *(Stability of Essential Oils: A Review,* Turek and Stintzing, 2013).

In particular, linalyl acetate is susceptible to oxidation following exposure to air and forms compounds, oxidation products, which determine a greater sensitizing power, significantly increasing the possibility of developing allergies. Linalool contained in the Lavender essential oil has a remarkable sensitizing power by inhalation or contact, causing allergies with a high probability.

In addition, the actives linalyl acetate and linalool are not soluble in aqueous environment or solvent.

The Applicant believes that it is not obvious to select at least one *carrier* or carrier agent suitable for promoting the physico-chemical stability of one or more extracts and active principles thereof within compositions, in particular compositions in solid form.

In this sense, there exists the need to find a composition suitable for chemically stabilizing and physically protecting the active principles of Lavender, in particular linalyl acetate and linalool, besides increasing the bioaccessibility and/or bioavailability thereof, preferably the enteric one.

In addition, there also exists the need to find compositions/formulations in solid form able to amplify the sedative/relaxing effect on the central nervous system and enhance sleep.

### SUMMARY OF THE INVENTION

The Applicant developed a powder composition comprising
a Lavender oil extract, comprising linalool and linalyl acetate, each in an amount comprised between 20% and 45% by weight based on the total weight of the extract,
an active and carrier agent consisting of rice okara,
for use in treating mild insomnia and/or mild anxiety.

A further object of the invention is the process for preparing the aforementioned powder composition, comprising the following steps
*i*) incorporating the lavender essential oil in the rice okara, and blending, preferably for a time comprised between 15 and 45 minutes;
*ii*) optionally, adding at least one anti-agglomerating food additive, and blending.

Finally, the Applicant developed an oral solid formulation comprising, as sole active ingredient, the powder composition alone, or the composition can be associated with at least one other active ingredient, preferably serving the same use for which the composition of the invention is used, anyway, in both cases in combination with suitable excipients and/or diluents.

### Advantages of the invention

The composition in solid form developed by the Applicant has, on the one hand, technological advantages related to the aspects of protection of the mucous membranes from gastric juices and from the oxidizing effect of the substances present in the gastrointestinal fluids that can degrade the actives of the Lavender extract; on the other hand, it has advantages in terms of nutraceutical use.

Specifically, with the composition of the invention it is possible to simultaneously achieve:
- an increase in solubility, chemical and physical stability in water and in the gastric environment of the Lavender extract and the active components of the essential oil contained therein, such as linalyl acetate and linalool. Such an effect is due to the presence of the carrier agent or *carrier* consisting of rice okara. Indeed, the carrier being discussed is able to protect the Lavender extract and the actives principles thereof, therefore increasing the bioavailability and/or bioaccessibility of the Lavender essential oil. Thereby, the passage of the aforementioned extract through the intestinal epithelium can be promoted, increasing its absorption at the intestinal level;
- an efficacy of use, mainly in the treatment of mild anxiety conditions, e.g. in case of stress, and/or mild insomnia; this is due to the presence of the actives contained in the Lavender oil. An increase in the stability, solubility and/or bioavailability of the Lavender essential oil results in an increase in the efficacy thereof for said purposes. In this sense, an amplified effect or an effect of reducing the mental stress levels and of enhancing the quality of sleep can be achieved.

### DESCRIPTION OF THE FIGURES

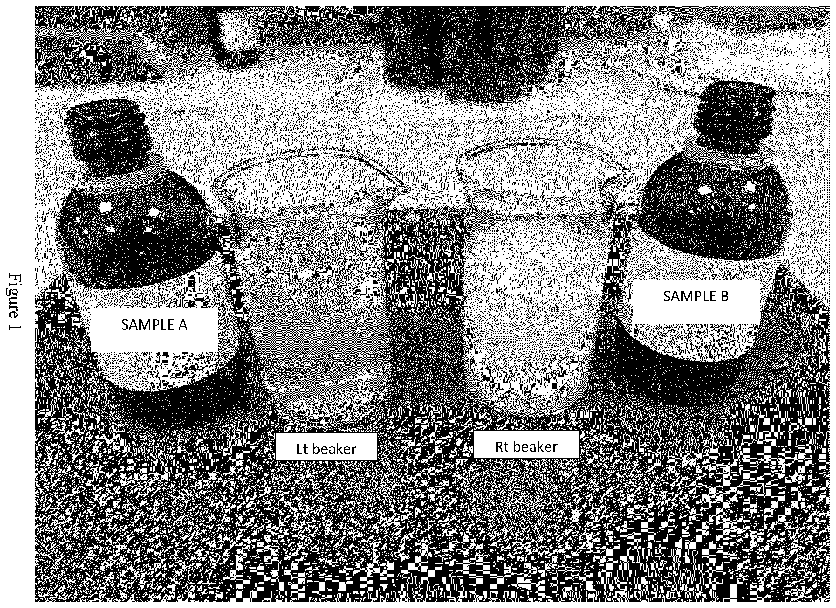

*Figure 1* - Photographic image of the visual comparison between the right beaker (Rt) containing Sample B according to the invention and the left beaker (Lt) containing the comparative Sample A (not according to the invention) referred to in Example 4.

### DETAILED DESCRIPTION OF THE INVENTION

Hereinafter, the Applicant will describe the invention in further detail. It should be noted that, although the structure has been organized in paragraphs and subparagraphs, the information contained in each paragraph or sub-paragraph are not isolated and can be possibly combined with those contained in other paragraphs or, more in general, with other information contained in the text of the Patent Application.

For the purposes of the present invention, the term "comprising" does not exclude the presence of further components which are not explicitly mentioned after this definition, while the term "consisting of" excludes this possibility.

It should be noted that, hereinafter, the Lavender oil extract is also referred to by the terms "Lavender oil" or "Lavender extract", both having the same meaning as the expression "Lavender oil extract".

### Composition

Preferably, the powder composition (or composition in solid form) comprises
a Lavender oil extract, comprising linalool in an amount comprised between 20% and 45% by weight based on the total weight of the extract, and linalyl acetate in an amount comprised between 25% and 47% by weight based on the total weight of the extract,
an active and carrier agent consisting of rice okara.

The Applicant points out that the Lavender oil extract and the rice okara are two active principles or agents.

Besides being an active, the rice okara is also a carrier agent or carrier.

Carrier agent or carrier means a substance or compound able to promote the chemical and/or physical stability of another substance or compound. In this case, the rice okara constitutes the carrier for the active Lavender oil extract.

Not only is the rice okara a solubilizer, but also a stabilizer with respect to the Lavender oil extract, especially to the active components of the essential oil contained therein, such as linalyl acetate and linalool, reducing or eliminating the phenomenon of dispersion due to their volatility, thus providing physical stability.

Preferably, the Lavender oil extract and the rice okara are in a weight ratio comprised between 1:0.5 and 1:7, preferably between 1:0.5 and 1:6.5, preferably between 1:0.5 and 1:5, preferably between 1:0.5 and 1:3, preferably between 1:1 and 1:3, preferably between 1:1 and 1:2.8, preferably between 1:1 and 1:2.8, preferably between 1:1 and 1:2.5, preferably between 1:2.5 and 1:2.8, preferably of 1:1 or 1:2.5, or 1:2.8 or 1:6.5. Alternatively, the Lavender oil extract and the rice okara are in a weight ratio comprised between 1:1 and 1:6.5.

It should be noted that this weight ratio is functional for the rice okara be able to act as a carrier and promote the stability of the Lavender oil extract within the composition of the invention.

A further advantage of the composition constituting the object of the invention lies in the fact that, despite containing an oily liquid substance, it is in the form of powder, preferably compressible powder, hence easily processable in solid forms, such as tablets, water-dispersible sachets, and not only in liquid-type oral formulations, such as micro- and nano-emulsions, soft capsules, liposomes and lipid nanoparticles.

According to a preferred embodiment, the composition of the invention is neither in micronized nor in nanoemulsion form; furthermore, it does not contain liposomes.

It should be noted that the Lavender oil extract can contain other actives, preferably lipophilic actives poorly or if at all soluble in water, such as camphor; preferably, the camphor is in an amount comprised between 0% and 4% by weight, preferably between 0% and 3% by weight, based on the total weight of the extract.

### Lavender oil extract

For the purposes of the present invention, extracts generally mean products of natural origin obtained from different types of plants and medicinal herbs. Various parts of the plant are useful for obtaining these products: leaves, flowers, fruits (pulp and/or peel), seeds, roots, rhizomes, bark, etc. They can be preparations in liquid, solid form (dry extracts) or of intermediate consistency (soft extracts), obtained by suitable extraction processes; the latter include the use of appropriate solvents, supercritical extraction, the use of maceration or percolation, or other suitable processes.

The Lavender oil extract is a liquid extract (or essential oil). Preferably, this extract is obtained by supercritical distillation or extraction, which is a well-known extraction technique, whereby the matter to be extracted is subjected to certain temperature and pressure conditions in the presence of a gas, generally carbon dioxide (CO₂).

Preferably, the Lavender oil extract is food grade, i.e. compliant with the specifications of food ingredients according to the *Codex Alimentarius* and European Regulation 925/2023, as well as European rules on pesticides, residual solvents, and the like.

Preferably, the Lavender oil extract is in an amount comprised between 1% and 90% by weight, preferably between 1% and 70% by weight, preferably between 1% and 55% by weight, preferably between 1% and 50% by weight, preferably between 3% and 50% by weight, preferably between 5% and 50% by weight, preferably between 8% and 50% by weight, preferably between 8% and 49% by weight, preferably between 8% and 25% by weight, preferably between 25% and 49% by weight, preferably of 8% or 25% or 49% by weight, based on the total weight of the composition.

Preferably, the Lavender belongs to a species and/or subspecies and/or variants and/or basionyms selected from the group consisting of: *Lavandula angustifolia Mill., Lavandula angustifolia subsp. pyrenaica (DC.) Guinea, Lavandula heterophylla Viv., Lavandula intermedia Emeric ex Loisel., Lavandula latifolia Medik, Lavandula stoechas L.,* preferably *Lavandula angustifolia Mill..*

The Lavender oil extract is preferably extracted from flowers and flowering tops of the aforementioned species and/or subspecies and/or variants and/or basionyms.

Preferably, the linalool included in the Lavender oil extract is in an amount comprised between 20% and 45% by weight, preferably between 20% and 47% by weight, preferably between 20% and 45% by weight, preferably between 25% and 40% by weight, based on the total weight of the extract.

Preferably, the linalyl acetate included in the Lavender oil extract is in an amount comprised between 20% and 45% by weight, preferably between 25% and 45% by weight, preferably between 30% and 45% by weight, based on the total weight of the extract.

### Rice okara

Rice okara is the white, floury pulp left over from making plant-based rice milk. In other words, rice okara corresponds to the solid leftover (or waste or by-product) resulting from the production of rice milk.

In this sense, rice okara is a food *waste.* The term "okara", used in the food context, generally falls within the context of plant-based drinks other than soy milk - such as, for example, almond okara or oat okara - with the intent of referring to the *waste* obtained following the production of these drinks. The rice okara is advantageously reused for the purposes of the invention in a circular economy perspective.

Preferably, the rice okara used for the purposes of the invention is rice okara powder.

In particular, the rice okara is obtained by the following method: after harvesting, the rice grain (from *Oryza sativa*) is macerated in water, then ground. Once ground, the ground rice grain undergoes enzymatic extraction followed by filtration. From the filtration, on the one hand rice okara, preferably wet rice okara, and on the other a rice drink (also commonly known by average consumers as "rice milk", taken as an alternative to cow's milk) are obtained. Rice okara is thus the solid waste resulting from the food production of these rice drinks; as mentioned above, it does not undergo any extraction, but rather drying and grinding processes.

Rice okara in powder form is obtained by the following method: wet rice okara, which can be obtained by the method described above, is cold-dried (or dried at low temperature, preferably of 40°C); this drying step aims at removing the aqueous component, which is recycled, as well as keeping intact the aromas and bioactive compounds naturally present, without adding additives, colorants or preservatives. Once dried, it undergoes grinding.

Preferably, the rice okara is in an amount comprised between 1% and 99% by weight, preferably between 1% and 80% by weight, preferably between 5% and 80% by weight, preferably between 10% and 80% by weight, preferably between 10% and 70% by weight, preferably between 20% and 70% by weight, preferably between 40% and 70% by weight, preferably between 45% and 70% by weight, preferably between 49% and 70% by weight, preferably between 50% and 70% by weight, preferably of 49% or 50% or 70% by weight, based on the total weight of the composition.

The rice okara combines the effect on mild anxiety and/or mild insomnia. Indeed, the rice okara is a source of amino acids (glutamic acid, tryptophan, glycine, arginine, phenylalanine tyrosine) useful as nutrients for regulating stress levels and sleep. The effect on the biosynthesis of neurotransmitters involved in the biological processes intended to regulate stress levels and sleep results therefrom.

Preferably, the rice okara contains an amount of protein comprised between 60% and 70% by weight, preferably between 60% and 68% by weight, preferably between 66% and 68% by weight, preferably of about 66% by weight, based on the total weight of rice okara.

Preferably, the rice okara contains an amount of total carbohydrate comprised between 14% and 18%, preferably between 15% and 17% by weight, preferably of about 16% by weight, based on the total weight of rice okara.

Preferably, the rice okara contains an amount of dietary fibre comprised between 4% and 7%, preferably between 5% and 7% by weight, preferably of about 6% by weight, based on the total weight of rice okara.

It should be noted that the rice okara contains a relatively high proportion of polysaccharides (carbohydrate), fibre and protein. This makes it represent the carrier or carrier agent of choice for the Lavender essential oil of the present invention.

It should be noted that rice okara cannot be considered as an equivalent of rice flour or glutenous rice flour. Indeed, the macromolecular composition of rice okara is significantly different from that of rice flour or glutenous rice flour: rice flour contains a prevalent amount of carbohydrate (79%) and only a minor amount of protein (7.3%). Rice flour has a protein content about 10 times less than rice okara. In this sense, according to the Applicant, rice flour/glutenous rice flour is not able to serve as an active and carrier agent. Further, rice okara and rice flour are not equivalent, since they also result from two different preparation processes.

The composition of the invention improves the stability of the Lavender essential oil and also promote the dispersion thereof in the rice okara. In particular, it is the rice okara that has a sequestering, hence protective, action on linalool and linalyl acetate contained in lavender essential oil. Besides that, the rice okara is also possibly able to promote the stabilization and the dispersion of *other and different* actives, which are insoluble in aqueous solvents, contained in the Lavender essential oil, such as camphor.

This phenomenon constitutes the premise of the stability of linalool and linalyl acetate in the powder composition, in the oral solid formulation - referred to in the following sections - and also in the digestive tract, resulting accordingly in a greater efficacy in promoting the physiological processes of sleep and serenity due to two factors:
- the maximum beneficial effect of Lavender essential oil and of the active principles thereof in enhancing sleep and reducing anxiety;
- the additional beneficial effect of rice okara, rich in protein (about 66%) and amino acids (tryptophan, arginine, glycine, glutamic acid, and others) involved in the physiology of sleep and tranquility.

It should be noted that the Applicant selected rice okara as the optimal *carrier* for Lavender oil extract. Other ingredients tested by the Applicant do not possess the same properties as rice okara for the purposes of the invention; for example, oat okara has a lower protein content (of about 43.3% by weight based on the total weight of the ingredient) than rice okara, which results in a less performing carrier function, in addition to containing a lower amount of amino acids for the regulation of stress levels and sleep.

### Anti-agglomerating food additive

Preferably, the powder composition constituting the object of the present invention comprises further at least one anti-agglomerating food additive.

Preferably, the at least one anti-agglomerating food additive is selected from the group consisting of: silicon dioxide and silicic acid salts, stearic acid fatty acid salts, talc, cellulose, microcrystalline cellulose, and other cellulose derivatives, calcium phosphates, mono-and diglycerides of fatty acids, carbonates and bicarbonates, and combinations thereof.

Preferably, the amount of the at least one anti-agglomerating food additive is comprised between 1% and 50% by weight, preferably between 1% and 45% by weight, preferably between 2% and 45% by weight, preferably between 2% and 42% by weight, preferably between 5% and 42% by weight, preferably between 2% and 5%, preferably of 2% or 5% or 42% by weight, based on the total weight of the composition.

Anti-agglomerating agents are useful for preventing the formation of agglomerates or aggregation of matter, e.g. in powder ingredients (agglomeration). These aggregation or agglomeration phenomena may be due, for example, to the absorption of water vapour contained in the atmosphere, or to the presence of fatty matter molecules that tend to bond to each other.

### Preparation process

The preparation process of the composition in solid form comprises the following steps:
*i*) incorporating the Lavender essential oil in the rice okara, and blending, preferably for a time comprised between 15 and 45 minutes, preferably between 15 and 30 minutes, preferably of 15 minutes or 30 minutes;
*ii*) optionally, adding at least one anti-agglomerating food additive, and blending, preferably for a time comprised between 15 and 30 minutes.

It should be noted that this process allows a tight aggregation between lavender essential oil and rice okara, which performs a sequestering activity, protecting the Lavender essential oil and the active principles contained therein; furthermore, it allows the same to be incorporated into solid formulations, such as tablets, capsules, sachets, single-dose powder.

### Oral solid formulation

The solid powder composition of the invention for use according to the present invention is included in an oral solid formulation comprising the same as active ingredient alone, or associated with at least one active ingredient for the same use, in combination with appropriate excipients and/or diluents.

According to *a first* preferred embodiment, the oral solid formulation contains:
the powder composition, as described above, as *sole* active ingredient,
in combination with suitable or appropriate excipients and/or diluents.

As an alternative to this first embodiment, a *second* preferred embodiment provides an oral solid formulation containing
the powder composition, as hitherto described, as active ingredient,
at least one other (or second) ingredient or active ingredient, different from the powder composition of the invention, preferably the latter serving the same use for which the powder composition of the invention is used,
in combination with appropriate excipients and/or diluents.

Preferably, suitable excipients and/or diluents for solid-type formulations are known to the person skilled in the art; for example, these can be selected from the group consisting of: bulking agents, glidants, lubricants, flow aids, disintegrants, flavourings, pH adjusters, sweeteners, gelatine, plasticizers, solvents, and combinations thereof.

Preferably, suitable excipients and/or diluents for the oral solid formulation are selected from the group consisting of: calcium phosphate, microcrystalline cellulose, hydroxypropylcellulose, cross-linked sodium carboxymethylcellulose, silicon dioxide, fatty acid magnesium salts, and combinations thereof.

Preferably, the at least one other or second active ingredient is a central nervous system sedative active and/or an anxiolytic and/or calming agent, for example for use in treating mild insomnia and/or mild anxiety.

Preferably, the at least one other or second active ingredient is in an amount comprised between 7% and 12% by weight, preferably between 8% and 10% by weight, preferably between 9% and 10% by weight, preferably of about 9.22% by weight, based on the total weight of the formulation.

Preferably, the at least one other or second active ingredient is selected from the group consisting of: Melatonin, Valerian, Lemon balm, GABA, plant extracts that promote relaxation, sleep, stress reduction, adaptogens, flavonoids, vitamins and minerals, and combinations thereof.

Preferably, the at least one other or second active ingredient is selected from the group consisting of: GABA, vitamins and combinations thereof. Preferably, vitamins are selected from the group consisting of: Vitamin D₃, Vitamin B₆, Vitamin B₁₂, and combinations thereof.

Preferably, the amount of the powder composition of the invention within the solid formulation is comprised between 45% and 60% by weight, more preferably between 50% and 55% by weight, still more preferably between 52% and 55% by weight, preferably of 53.7% or 54% by weight, based on the total weight of the formulation.

Preferably, the Lavender oil extract is in an amount comprised between 5% and 10% by weight, preferably between 5% and 8% by weight, preferably between 6% and 8%, preferably of about 7.3% by weight, based on the total weight of the formulation.

Preferably, rice okara is in an amount comprised between 35% and 50% by weight, preferably between 40% and 50% by weight, preferably between 45% and 48% by weight, preferably of about 46.5% by weight, based on the total weight of the formulation.

It should be noted that the amount of the powder composition of the invention within the solid formulation is such regardless of whether other actives are present or not.

Preferably, the solid formulation can be in the form of tablet, capsule, powder or orodispersible or water-dispersible granulate, stick, comprising the composition in powder or granulate form therein.

Preferably, suitable excipients and/or diluents for solid-type formulations are known to the person skilled in the art; for example, these can be selected from the group consisting of: bulking agents, thickeners, glidants, exfoliating agents, lubricants, flow aids, disintegrants, flavourings, pH adjusters, sweeteners, gelatine, plasticizers, solvents, silicon derivatives, and combinations thereof.

For example, suitable excipients and/or diluents included in the solid-type formulation are selected from the group consisting of: microcrystalline cellulose, modified cellulose derivatives (e.g., hydroxypropylcellulose), dibasic calcium phosphate, silicon dioxide, silicon-derived powders, magnesium stearate, and mixtures thereof.

Preferably, the oral solid formulation is in the form of nutraceutical (or nutraceutical formulation), or in the form of food supplement, or still in the form of Food for Special Medical Purposes (FSMP).

Nutraceutical means a food derivative to which, besides the basic nutritive value, the capability of "optimizing" physiological functions is attributed.

Food supplement means a formulation falling under the definition encompassed by the Directive 2002/46/EC and the subsequent amendments. In this legislation, food supplements are defined as: "dietary products designed to integrate the normal diet that are a concentrated source of nutrients, such as vitamins and minerals, or of other substances with nutritional or physiological effects, especially but not only amino acids, essential fatty acids, fibres and plant-based extracts, either mono- or multi-compounds, in pre-dosed form".

Foods for Special Medical Purposes (or FSMPs) mean food products specifically processed or formulated for the dietary management of patients with specific nutritional needs, including infants, to be used "under medical supervision". FSMPs are intended for the exclusive or partial feeding of patients having a limited or altered ability to take, digest, absorb, metabolize or eliminate ordinary foods or certain nutrients contained therein, or having specific nutritional needs determined by clinical conditions. FSMPs differ from other foodstuffs and food supplements due to some characteristics, such as: they require medical supervision; they can replace - totally or partially - the normal diet; they are indicated for specific pathological conditions. As for the composition, FSMPs are regulated by the Regulation 609/13.

### Usage

Preferably, the composition of the invention is for use in treating mild insomnia and/or mild anxiety.

Mild insomnia means the disorder of insomnia of a mild degree or intensity. The symptoms related to insomnia can be nocturnal, such as difficulty falling asleep, frequent or prolonged awakenings at night, early morning awakenings, or diurnal, feeling of fatigue and low energy, difficulty paying attention, concentrating and remembering, mood disorders, difficulty working or studying.

Mild anxiety means a state of worry of variable duration or frequency. It is caused by a feeling of agitation for events or everyday problems and is characterized by the thought of being inadequate to manage them. Anxiety disorders arise above all in difficult situations related to everyday life (work, family, everyday tasks or financial problems). Mild anxiety causes a range of symptoms, such as restlessness, constant worry, disturbed sleep and bad mood, which can impact on the quality of life.

The powder composition is useful for promoting relaxation in case of stress and/or sleep, and/or to assist in the normalization of mood tone.

Preferably, the oral solid formulation comprising the composition described in the previous sections is for use in treating mild insomnia and/or mild anxiety, as well.

Preferably, the composition or oral solid formulation of the invention is for use in treating insomnia and/or anxiety disorder.

Insomnia is a sleep disorder that, if persistent and clinically significant, is recognized as a medical condition (DSM-5 (Diagnostic and Statistical Manual of Mental Disorders); CSD-3 (International Classification of Sleep Disorders)).

Anxiety disorder is a pathology (DSM-5 (Diagnostic and Statistical Manual of Mental Disorders)) that, if constant or recurrent, is difficult to control, causes clinical symptoms and interferes with work or social sphere.

### EXAMPLES

Below, the Applicant provides examples for merely illustrative, but non-limiting purposes.

### Example 1 - Composition in solid form

| **Ingredient** | % |
|---|---|
| Lavender essential oil (from *Lavandula angustifolia*), titrated at 30-45% in linalyl acetate and at 25-40% in linalool | 8 |
| Rice okara | 50 |
| Anti-agglomerating additives | 42 |

### Preparation method of the composition according to Example 1

1. Mixing rice okara and additives.
2. Incorporating the essential oil into the mixture from point 1. Using a planetary kneader or a whisk or ribbon mixer for a processing time of 30 minutes.

### Example 2 - Composition in solid form

| **Ingredient** | % |
|---|---|
| Lavender essential oil (from *Lavandula angustifolia*), titrated at 30-45% in linalyl acetate and at 25-40% in linalool | 25 |
| Rice okara | 70 |
| Anti-agglomerating additives | 5 |

### Preparation method of the composition according to Example 2

1. Incorporating the essential oil into the rise okara from point. Uusing a planetary kneader or a whisk or ribbon mixer for a processing time of 15 minutes.
2. Adding the additives and incorporating by mixing with the same equipment for further 30 minutes.

### Example 3 - Composition in solid form

| **Ingredient** | **%** |
|---|---|
| Lavender essential oil (from *Lavandula angustifolia*), titrated at 30-45% in linalyl acetate and at 25-40% in linalool | 49 |
| Rice okara | 49 |
| Anti-agglomerating additives | 2 |

### Preparation method of the composition according to Example 3

1. Mixing rice okara and additives
2. Incorporating the essential oil into the mixture from point 1. Using a planetary kneader or a whisk or ribbon mixer for a processing time of 30 minutes.

### Example 4 - Experimental test to prove the sequestering effect of rice okara on Lavender essential oil.

### Preparation of Sample A (Comparative sample)

**Table 1**

| ***Sample A*** | |
|---|---|
| *Components* | *Amount (grams)* |
| Lavender essential oil | 2 g |
| Water | 85.2 g |

### Solubilizing under vortex stirring, centrifuging and filtering

2 g of Lavender essential oil were added to 85.2 ml of demineralized water, placed under stirring for 10 minutes, centrifuged at 4000 rpm for 5 minutes, and filtered with filter paper in a transparent beaker.

### Preparation of Sample B (Sample according to the invention)

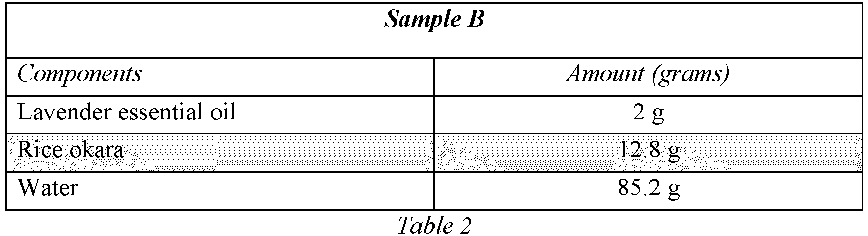

### Solubilizing under vortex stirring, centrifuging and filtering

14.8 g of a composition comprising Lavender essential oil and rice okara (white in colour), in turn containing 2 g of Lavender essential oil and 12.8 g of rice okara, were added to 85.2 ml of demineralized water, placed under stirring for 10 minutes, centrifuged at 4000 rpm for 5 minutes, and filtered with filter paper into a transparent beaker.

The appearance of the two pre-filtration samples is compared (refer to Figure 1).

From the visual comparison, it is evident that the combination of Lavender essential oil and rice okara generates a milky and *homogeneous* solution (right beaker in Figure 1), indicating that some water-soluble components of the rice okara are solubilized in water in a homogeneous manner; the Lavender essential oil as is in water generates, in contrast, a slightly cloudy and *only partially* homogeneous solution, a sign that the essential oil is little or not at all soluble in water (left beaker in Figure 1).

### 4.1 Measurement of linalool and linalyl acetate content in Samples A and B

Further, Samples A and B of Example 4 are tested to examine the linalool and linalyl acetate content by gas chromatography. The acetate and linalool content was analytically checked in both filtered samples, providing the following results.

**Table 3**

| | *linalool content (ppm)* | *linalyl acetate content (ppm)* |
|---|---|---|
| *Sample A* | 6870 ppm | 8800 ppm |
| *Sample B* | 1030 ppm | 842 ppm |

The linalool concentration in the filtrate is reduced *by more than a sixth* in Sample B (compound Lavender essential oil + rice okara), compared to Sample A (Lavender essential oil dispersed in water).

The linalyl acetate concentration in the filtrate is reduced *by more than a tenth* in Sample B (compound Lavender essential oil + rice okara) compared to Sample A (Lavender essential oil dispersed in water).

This demonstrates a real sequestering and protective effect of rice okara on the active principles of Lavender: linalool and linalyl acetate.

### Example 5 - Formulation (tablet) comprising the composition in solid form of the invention

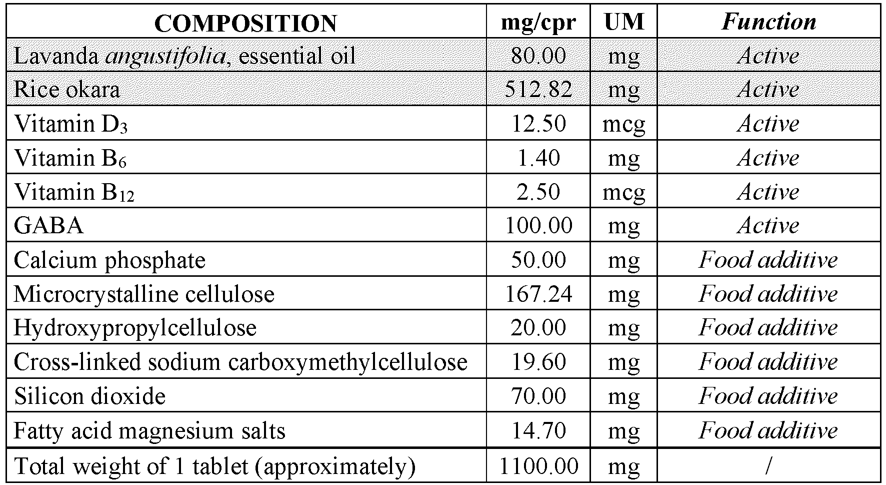

### LITERATURE

1. OECD SIDS LINALYL ACETATE
2. Goutam Mondal 1, Olivia R. Dale 1, Yan-Hong Wang 1, Shabana I. Khan 1,2, Ikhlas A. Khan 1,2 and Charles R. Yates. In Vitro Metabolism and CYP-Modulating Activity of Lavender Oil and Its Major Constituents. Molecules 2023, 28, 755.
3. Martha J. Greenberg, Jason T. Slyer. Effectiveness of Silexan oral lavender essential oil compared to inhaled lavender essential oil aromatherapy for sleep in adults: a systematic review. 2018 THE JOANNA BRIGGS INSTITUTE
4. Wuan Shuen Yap1, Anton V. Dolzhenko 1,2, Zahraa Jalal3*, Muhammad Abdul Hadi 3 & Tahir Mehmood Khan1. Efficacy and safety of lavender essential oil (Silexan) capsules among patients suffering from anxiety disorders: A network meta-analysis. Scientific Reports (2019) 9:18042
5. Walter E. Müller a,*, Giacomo Sillani a, Anita Schuwald a, Kristina Friedland. Pharmacological basis of the anxiolytic and antidepressant properties of Silexan®, an essential oil from the flowers of lavender. Neurochemistry International 143 (2021) 104899
6. Kasper, WE Müller, HP Volz, HJ Möller, E Koch & A Dienel. Silexan in anxiety disorders: clinical data and pharmacological background. ISSN: 1562-2975 (Print) 1814-1412
7. Cinzia Cimino 1 , Oriana Maria Maurel 2, Teresa Musumeci 1 , Angela Bonaccorso 1 , Filippo Drago 2, Eliana Maria Barbosa Souto 3,4, Rosario Pignatello 1 and Claudia Carbone. Essential Oils: Pharmaceutical Applications and Encapsulation Strategies into Lipid-Based Delivery Systems. Pharmaceutics 2021, 13, 327.
8. Z. Dajic Stevanovic, E. Sieniawska, K. Glowniak., N Obradovic and I. Pajic-Lijakovic. Natural Macromolecules as Carriers for Essential Oils: From Extraction to Biomedical Application. Frontiers in Bioengineering and Biotechnology June 2020, Volume 8, Article 563.
9. Fernando, J. Rice as a Source of Fibre. Rice Res 2013, 1:2
10. Elsa Trinovita 1, Fadlina Chany Saputri 2,* and Abdul Mun'im. Potential Gastroprotective Activity of Rice Bran (Oryza sativa L.) Extracted by Ionic Liquid-Microwave Assisted Extraction against Ethanol-Induced Acute Gastric Ulcers in Rat Model. Sci. Pharm. 2018, 86, 35;
11. HC Yadav and Vandana. Functional and nutritional qualities of okara and food industry applications. The Pharma Innovation Journal 2023; 12(9): 2648-2650
12. Fakhar Islam1 | Yuosra Amer Ali2 | Ali Imran1 | Muhammad Afzaal1 | Syeda Mahvish Zahra3,4 | Maleeha Fatima5 | Farhan Saeed1 | Ifrah Usman1 |Umber Shehzadi2 | Shilpa Mehta6 | Mohd Asif Shah7. Vegetable proteins as encapsulating agents: Recent updates and future perspectives. Food Sci Nutr. 2023;11:1705-1717

## Claims

1. A powder composition comprising
a Lavender oil extract, comprising linalool and linalyl acetate, each in an amount comprised between 20% and 45% by weight based on the total weight of the extract,
an active and carrier agent consisting of rice okara,
for use in treating mild insomnia and/or mild anxiety.

2. The composition for use according to claim 1, wherein the Lavender oil extract and the rice okara are in a weight ratio comprised between 1:0.5 and 1:7.

3. The composition for use according to claim 1 or 2, wherein the Lavender oil extract is in an amount comprised between 1% and 90% by weight based on the total weight of the composition.

4. The composition for use according to any one of claims from 1 to 3, wherein the rice okara is in an amount comprised between 1% and 99% by weight based on the total weight of the composition.

5. The composition for use according to any one of claims from 1 to 4, wherein the Lavender belongs to a species and/or subspecies and/or variants and/or basionyms selected from the group consisting of: *Lavandula angustifolia Mill., Lavandula angustifolia subsp. pyrenaica (DC.) Guinea, Lavandula heterophylla Viv., Lavandula intermedia Emeric ex Loisel., Lavandula latifolia Medik, Lavandula stoechas L..*

6. The composition for use according to any one of claims from 1 to 5, further comprising at least one anti-agglomerating food additive.

7. The composition for use according to claim 6, wherein the at least one anti-agglomerating food additive is selected from the group consisting of: silicon dioxide and silicic acid salts, stearic acid fatty acid salts, talc, cellulose, microcrystalline cellulose, and other cellulose derivatives, calcium phosphates, mono-and diglycerides of fatty acids, carbonates and bicarbonates, and combinations thereof.

8. The composition for use according to any one of claims from 1 to 7, in the form of
an oral solid formulation containing
the powder composition according to any one of claims from 1 to 7, as *sole* active ingredient,
in combination with suitable excipients and/or diluents,
*or*
an oral solid formulation containing
the powder composition according to any one of claims from 1 to 7, as active ingredient,
at least one second active ingredient, different from the powder composition, wherein the at least one second active ingredient is a central nervous system sedative and/or an anxiolytic,
in combination with appropriate excipients and/or diluents.

9. The composition for use according to claim 8, wherein said oral solid formulation is in the form of nutraceutical, food supplement or Food for Special Medical Purposes.

10. A process for preparing the powder composition according to any one of claims from 1 to 9, comprising the following steps:
*i*) incorporating the lavender essential oil in the rice okara, and blending, preferably for a time comprised between 15 and 45 minutes;
*ii)* optionally, adding at least one anti-agglomerating food additive, and blending.
